# EUROPEAN PATENT SPECIFICATION

(11) **EP 4 563 191 B1**
(45) Date of publication and mention of the grant of the patent: **03.06.2026**
(21) Application number: 24215715.4
(22) Date of filing: 27.11.2024
(51) Int. Cl.: A61N 1/372, A61B 17/34, A61M 25/00, A61N 1/375, A61N 1/05

(54) **BIOSTIMULATOR TRANSPORT SYSTEM HAVING ROTATIONALLY COUPLED SHAFTS**
BIOSTIMULATORTRANSPORTSYSTEM MIT DREHGEKOPPELTEN WELLEN
SYSTÈME DE TRANSPORT DE BIOSTIMULATEUR AYANT DES ARBRES COUPLÉS EN ROTATION

(30) Priority: 30.11.2023 US 202363604833 P; 22.11.2024 US 202418957570
(43) Date of publication of application: 04.06.2025
(73) Proprietor: Pacesetter, Inc., Sylmar, CA 91342 (US)
(72) Inventor: Arnar, Bernhard, Sylmar, CA 91342 (US); Senarith, Patrick, Sylmar, CA 91342 (US); Jackson, Aaron, Sylmar, CA 91342 (US)
(74) Representative: Barker Brettell Sweden AB

(56) References cited:
- US-A1- 2012 095 539
- US-A1- 2016 279 423
- US-A1- 2023 173 285

## Description

### TECHNICAL FIELD

The present disclosure relates to biostimulators and related biostimulator systems. More specifically, the present disclosure relates to leadless biostimulators and related systems useful for septal pacing.

### BACKGROUND

Cardiac pacing by an artificial pacemaker provides an electrical stimulation of the heart when its own natural pacemaker and/or conduction system fails to provide synchronized atrial and ventricular contractions at rates and intervals sufficient for a patient's health. Such antibradycardial pacing provides relief from symptoms and even life support for hundreds of thousands of patients. Cardiac pacing may also provide electrical overdrive stimulation to suppress or convert tachyarrhythmias, again supplying relief from symptoms and preventing or terminating arrhythmias that could lead to sudden cardiac death.

Leadless cardiac pacemakers incorporate electronic circuitry at the pacing site and eliminate leads, thereby avoiding shortcomings associated with conventional cardiac pacing systems. Leadless cardiac pacemakers can be anchored at the pacing site, e.g., in a right ventricle and, for dual-chamber pacing, in a right atrium, by an anchor. A delivery system can be used to deliver the leadless cardiac pacemakers to the target anatomy.

Cardiac pacing of the His-bundle is clinically effective and advantageous by providing a narrow QRS affecting synchronous contraction of the ventricles. His-bundle pacing in or near a membranous septum of a heart, however, has some drawbacks. The procedure is often long in duration and requires significant fluoroscopic exposure. Furthermore, successful His-bundle pacing cannot always be achieved. Pacing thresholds are often high, sensing is challenging, and success rates can be low.

Pacing at the left bundle branch area (LBBAP) is an alternative to His-bundle pacing. LBBAP involves pacing past the His-bundle toward the right ventricular apex. More particularly, a pacing site for LBBAP pacing is typically below the His-bundle, on the interventricular septal wall. LBBAP can prevent pacing induced cardiomyopathy in a bradycardia patient population. LBBAP can also be an effective alternative to cardiac resynchronization therapy in treating heart failure patients. To achieve optimal results, the pacing site for physiological LBBAP can be high on the interventricular septal wall, in the region close to the tricuspid valve and pulmonary artery outflow track. Furthermore, the pacing site may be at a depth of up to 1.5 cm within the septal wall.

US 2016/279423 A1, US 2012/095539 A1 and US 2023/173285A1 are prior art documents showing conventional implantable medical device (IMD) or leadless biostimulator delivery systems.

### SUMMARY

A significant challenge associated with delivering a leadless cardiac pacemaker to the left bundle branch area pacing (LBBAP) pacing site is engaging the septal wall at an optimal location and angle. More particularly, existing leadless pacemakers may not fit, or may interfere with heart structures, when placed at the optimal pacing site for LBBAP. Existing leadless pacemakers have bodies that are long and rigid and, when implanted at the interventricular septal wall, could extend into contact with the cardiac tissue of a ventricular free wall or the tricuspid valve during contraction of the heart. Moving the existing leadless pacemakers further down the septal wall can cause the pacemaker to engage the distal fascicles and/or fibers, rather than engaging bundle branches directly, which can result in suboptimal activation of the conduction system and reduced battery life. Furthermore, a proximal end of the existing leadless pacemakers may flail within the heart chamber as the heart beats, causing cyclical contact with adjacent heart structures. Such contact could interfere with heart function. Thus, there is a need for a leadless biostimulator transport system that can deliver a leadless cardiac pacemaker to engage the interventricular septal wall at an appropriate location and angle such that the biostimulator can pace the LBB directly for improved conduction system capture and battery life, without interfering with adjacent structures of the heart.

The present invention is defined in the independent claim. Further embodiments of the invention are defined in the dependent claims.

A biostimulator transport system is described. In an embodiment, the biostimulator transport system includes an input shaft and an output shaft. The input shaft extends distally, e.g., in an antegrade direction, to an input gear. The output shaft extends proximally, e.g., in a retrograde direction, from an output gear to a biostimulator coupling. The output gear is rotationally coupled to the input gear such that rotation of the input shaft drives rotation of the biostimulator coupling.

A biostimulator system is described. In an embodiment, the biostimulator system includes a biostimulator mounted on the biostimulator transport system. An exemplary, non-claimed method of delivering the biostimulator to a target anatomy using the biostimulator transport system is also described.

The above summary does not include an exhaustive list of all aspects of the present invention. It is contemplated that the invention is defined by the appended claims.

### BRIEF DESCRIPTION OF THE DRAWINGS

The features of the invention are set forth with particularity in the claims that follow. A better understanding of the features and advantages of the present invention will be obtained by reference to the following detailed description that sets forth illustrative embodiments, in which the principles of the invention are utilized, and the accompanying drawings.
FIG. 1 is a diagrammatic cross section of a patient heart illustrating an example implantation of a biostimulator in a target anatomy, in accordance with an embodiment.
FIG. 2 is a perspective view of a biostimulator system, in accordance with an embodiment.
FIG. 3 is a side view of a biostimulator system in an undeployed state, in accordance with an embodiment.
FIG. 4 is a side view of a biostimulator system in a deployed state, in accordance with an embodiment.
FIG. 5 is a side view of a biostimulator deployed through a window of a biostimulator transport system, in accordance with an embodiment.
FIG. 6 is a side view of a biostimulator transport system having fixed angle gears in an undeployed state, in accordance with an embodiment.
FIG. 7 is a side view of a biostimulator transport system having fixed angle gears in a deployed state, in accordance with an embodiment.
FIG. 8 is a side view of a biostimulator transport system having adjustable angle gears in an undeployed state, in accordance with an embodiment.
FIG. 9 is a side view of a biostimulator transport system having adjustable angle gears in a deployed state, in accordance with an embodiment.
FIG. 10 is a side view of a biostimulator transport system having a shaft actuator in an undeployed state, in accordance with an embodiment.
FIG. 11 is a side view of a biostimulator transport system having a shaft actuator in a deployed state, in accordance with an embodiment.
FIG. 12 is a cross-sectional view of gears contained in a boot of a biostimulator transport system, in accordance with an embodiment.
FIG. 13 is a perspective view of meshed spur gears of a biostimulator transport system, in accordance with an embodiment.
FIG. 14 is a perspective view of meshed spherical gears of a biostimulator transport system, in accordance with an embodiment.
FIG. 15 is a side view of a micromotor of a biostimulator transport system driving a biostimulator, in accordance with an embodiment.
FIG. 16 is a side view of a micromotor driving meshed gears of a biostimulator transport system, in accordance with an embodiment.
FIG. 17 is a side view of a micromotor of a biostimulator transport system driving a biostimulator, in accordance with an embodiment.
FIG. 18 is a flowchart of an exemplary, non-claimed method of delivering a biostimulator using a biostimulator transport system having rotationally coupled shafts, in accordance with an embodiment.
FIGS. 19-20 are pictorial views of operations of an exemplary, non-claimed method of delivering a biostimulator using a biostimulator transport system having rotationally coupled shafts, in accordance with an embodiment.
FIG. 21 is a perspective view of a biostimulator transport system including a biostimulator coupling having a universal joint, in accordance with an embodiment.
FIG. 22 is a perspective view of a biostimulator coupling having a universal joint, in accordance with an embodiment.
FIG. 23 is a cross-sectional view of a biostimulator coupling having a universal joint in a first state, in accordance with an embodiment.
FIG. 24 is a cross-sectional view of a biostimulator coupling having a universal joint in a second state, in accordance with an embodiment.

### DETAILED DESCRIPTION

Embodiments describe a biostimulator and a biostimulator system for pacing, e.g., septal pacing. The biostimulator may, however, be used in other applications, such as deep brain stimulation. Thus, reference to the biostimulator as being a cardiac pacemaker for pacing, or septal pacing, is not limiting.

In various embodiments, description is made with reference to the figures. However, certain embodiments may be practiced without one or more of these specific details, or in combination with other known methods and configurations. In the following description, numerous specific details are set forth, such as specific configurations, dimensions, and processes, in order to provide a thorough understanding of the embodiments. In other instances, well-known processes and manufacturing techniques have not been described in particular detail in order to not unnecessarily obscure the description. Reference throughout this specification to "one embodiment," "an embodiment," or the like, means that a particular feature, structure, configuration, or characteristic described is included in at least one embodiment. Thus, the appearance of the phrase "one embodiment," "an embodiment," or the like, in various places throughout this specification are not necessarily referring to the same embodiment. Furthermore, the particular features, structures, configurations, or characteristics may be combined in any suitable manner in one or more embodiments.

The use of relative terms throughout the description may denote a relative position or direction. For example, "distal" may indicate a first direction along a longitudinal axis of a biostimulator. Similarly, "proximal" may indicate a second direction opposite to the first direction. Such terms are provided to establish relative frames of reference, however, and are not intended to limit the use or orientation of a biostimulator to a specific configuration described in the various embodiments below.

In an aspect, a biostimulator transport system includes rotationally coupled shafts to drive rotation of a biostimulator. More particularly, the biostimulator transport system includes an input shaft rotationally coupled to an output shaft via a joint. For example, the joint may include a meshing gear joint. The meshing gear joint can include an input gear coupled to the input shaft and an output gear coupled to the output shaft. The biostimulator may be mounted on a biostimulator coupling at an end of the output shaft. The biostimulator coupling may be proximal to the gears in the gear train. The biostimulator may therefore be directed proximally from the gears, e.g., in a retrograde direction relative to a direction of delivery into a target anatomy. When the biostimulator transport system is delivered into the target anatomy and the biostimulator is deployed to direct a helical fixation element of the biostimulator toward a target tissue, the rotationally coupled shafts can therefore form a V shape with the biostimulator directed proximally in the retrograde direction. The gear train may operate to rotate and anchor the biostimulator in the target tissue, e.g., a septal wall, with a proximal end of the biostimulator being located distally within the target anatomy, e.g., in a ventricular apical space.

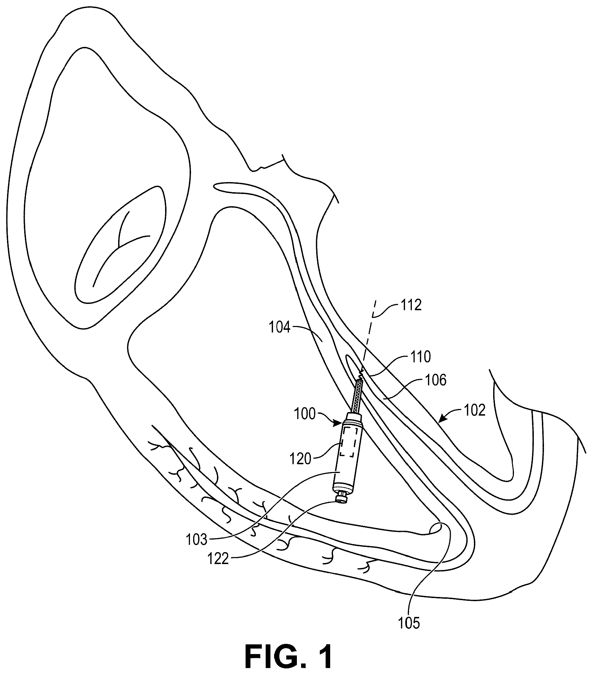

Referring to FIG. 1, a diagrammatic cross section of a patient heart illustrating an example implantation of a biostimulator in a target anatomy is shown in accordance with an embodiment. The diagram shows a biostimulator 100 attached to a patient heart 102 with a body 103 of the biostimulator positioned toward a ventricular apex. A leadless biostimulator system, e.g., a cardiac pacing system, can include the biostimulator 100. The biostimulator 100 can be implanted in the patient heart 102, and can be leadless (and thus, may be a leadless cardiac pacemaker). The biostimulator 100 can be placed in a cardiac chamber, such as a right atrium and/or right ventricle of the heart 102, or attached to an inside or outside of the cardiac chamber. For example, the biostimulator 100 can be attached to one or more of an interventricular septal wall 104 or a ventricular apex 105 of the heart 102. More particularly, the biostimulator 100 can be delivered to an interventricular septum, and one or more elements, such as a helical fixation element 110, can pierce the interventricular septal wall 104 of the septum to engage and anchor the biostimulator 100 to a target tissue 106. The body 103 of the biostimulator 100 can be directed toward the ventricular apex 105. More particularly, the biostimulator 100 can extend along a longitudinal axis 112, and the body 103 of the biostimulator can extend distally from the helical fixation element 110, which is positioned in a left bundle branch area, to a proximal end of the biostimulator that can mount on a biostimulator transport system, as described below. Accordingly, the longitudinal axis 112 can be oblique or parallel to a wall surface of the septal wall 104 when the helical fixation element 110 is affixed to the septal wall. For example, a proximal end of the body 103 may be located at the apex, or nearer to the apex than a distal end of the body.

When the biostimulator 100 is delivered to and plunged into the septum of the heart 102, the helical fixation element 110 may be positioned for deep septal pacing at a target anatomy, such as at a bundle branch in the septum. For example, an electrode of the biostimulator 100, which may include the helical fixation element 110 can be positioned at a left bundle branch in the septum. The biostimulator 100 may deliver pacing impulses through the pacing electrode to the target anatomy. Accordingly, the pacing electrode can be located to effectively probe and pace the target anatomy, while the body 103 can be placed in a safe and non-obstructive location within the heart chamber.

The biostimulator 100 can be a leadless cardiac pacemaker that can perform cardiac pacing and that has many of the advantages of conventional cardiac pacemakers while extending performance, functionality, and operating characteristics. In a particular embodiment, the biostimulator 100 can use two or more electrodes located on or within the body 103 of the biostimulator 100 for pacing the cardiac chamber upon receiving a triggering signal from at least one other device within the patient anatomy. The two or more electrodes of the biostimulator 100 can include an electrode of the helical fixation element 110 that acts as an active electrode. The electrodes can deliver pacing pulses to target anatomies, such as bundle branches within the septum of the heart 102, to perform pacing, e.g., deep septal pacing, and optionally, can sense electrical activity from the muscle. The electrodes may also communicate bidirectionally with at least one other device within or outside the patient anatomy.

The body 103 can contain a primary battery to provide power for pacing, sensing, and communication, which may include, for example, bidirectional communication. The body 103 can optionally have an electronics compartment 120 (shown by hidden lines) to hold circuitry adapted for different functionality. For example, the electronics compartment 120 can contain pacing circuitry for sensing cardiac activity from the electrodes, for receiving information from at least one other device via the electrodes, for generating pacing pulses for delivery to tissue via the electrode(s), or other circuitry. Accordingly, the pacing circuitry can be electrically connected to the electrode(s). The electronics compartment 120 may contain circuits for transmitting information to at least one other device via the electrodes and can optionally contain circuits for monitoring device health. The circuitry of the biostimulator 100 can control these operations in a predetermined manner. In some implementations of a cardiac pacing system, cardiac pacing is provided without a pulse generator located in the pectoral region or abdomen, without an electrode-lead separate from the pulse generator, without a communication coil or antenna, and without an additional requirement of battery power for transmitted communication.

The helical fixation element 110 can retain the body 103 within a target tissue 106. For example, the fixation element 110 can include a helix, e.g., a helically wound wire having a sharpened tip, to pierce and screw into the target tissue 106. The target tissue 106 may be a predetermined portion of the target anatomy, such as a left bundle branch in the septal wall.

In an embodiment, the biostimulator 100 includes an attachment feature 122 to allow a biostimulator transport system to grasp and retain the biostimulator 100 in vivo. The attachment feature 122 may be mounted on a proximal body end of the body 103. The attachment feature 122 may have a graspable shape, such as a button, a hook, a knob, or another shape that has a varying cross-sectional profile. For example, the attachment feature 122 can have a head connected to the body 103 by a neck portion. The head may have a larger cross-sectional dimension than the neck. A loop of a snare device may be placed around the neck and cinched to grab the attachment feature 122 and hold the body 103 relative to the biostimulator transport system.

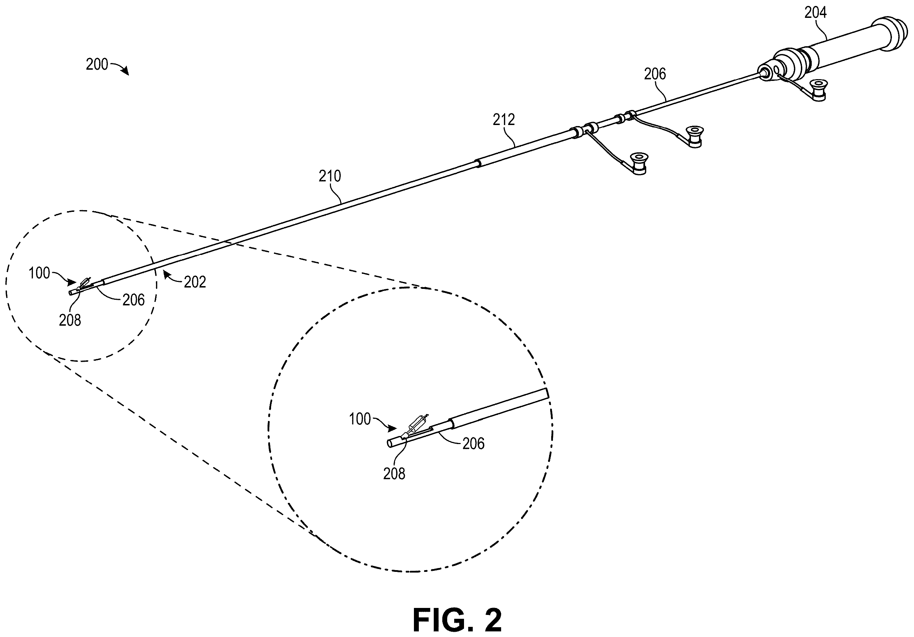

Referring to FIG. 2, a perspective view of a biostimulator system is shown in accordance with an embodiment. Leadless pacemakers or other leadless biostimulators can be delivered to or retrieved from a patient using delivery or retrieval systems. The leadless biostimulator system can include delivery or retrieval systems, which may be catheter-based systems used to carry a leadless biostimulator intravenously to or from a patient anatomy. The delivery or retrieval systems may be referred to collectively as transport systems, or biostimulator transport systems.

A biostimulator system 200 can include a biostimulator transport system 202. The biostimulator 100 can be attached, connected to, or otherwise mounted on the biostimulator transport system 202. For example, the biostimulator 100 can be mounted on an output shaft of the biostimulator transport system 202, as described below. The biostimulator 100 is thereby advanced intravenously into or out of the heart 102.

The biostimulator transport system 202 can include a handle 204 to control movement and operations of the transport system from outside of a patient anatomy. One or more elongated members can extend distally from the handle 204. For example, an inner sheath 206 can extend distally from the handle 204. The inner sheath 206 can extend to a distal end of the transport system. In an embodiment, the biostimulator 100 is deployable through a window 208 of the inner sheath 206.

The biostimulator transport system 202 can include an outer sheath 210. The outer sheath 210 can cover the biostimulator 100 and/or inner sheath 206 during delivery and implantation. The outer sheath 210 can extend over, and be longitudinally movable relative to, the inner sheath 206. The biostimulator transport system 202 may also include an introducer sheath 212 that can extend over, and be longitudinally movable relative to, the outer sheath 210. The introducer sheath 212 can cover a distal end of the outer sheath 210, the inner sheath 206, and the biostimulator 100 as those components are passed through an access device into the patient anatomy.

Several components of the biostimulator transport system 202 are described above by way of example. It will be appreciated, however, that the biostimulator transport system 202 may be configured to include additional or alternate components. More particularly, the biostimulator transport system 202 may be configured to deliver and/or retrieve the biostimulator 100 to or from the target anatomy. Delivery and/or retrieval of the biostimulator 100 can include retaining the biostimulator during transport to the target anatomy and rotation of the biostimulator during implantation of the biostimulator at the target anatomy. Accordingly, the biostimulator transport system 202 can incorporate features to retain and rotate the biostimulator 100.

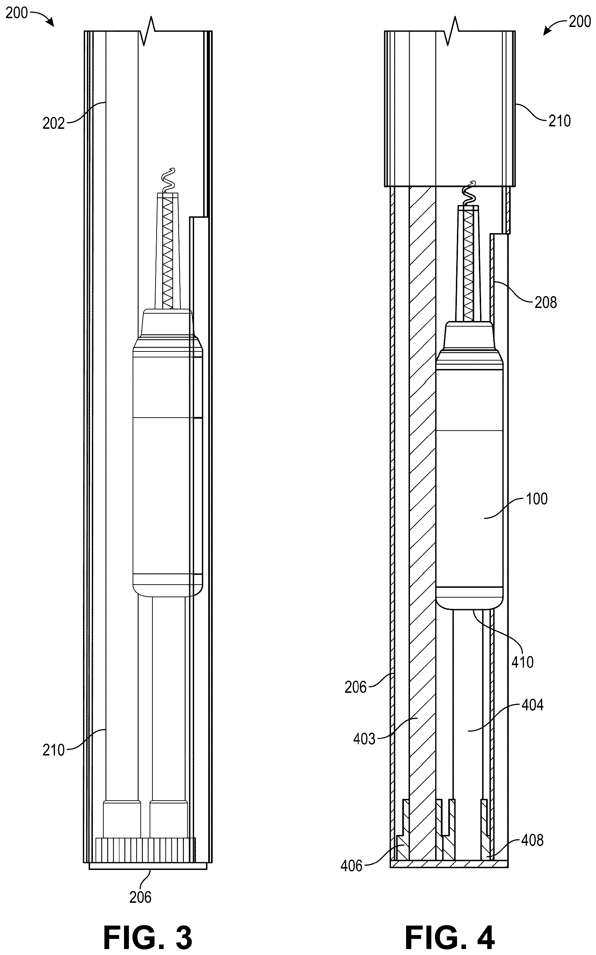

Referring to FIG. 3, a side view of a biostimulator system in an undeployed state is shown in accordance with an embodiment. The biostimulator transport system 202 can include a dual-sheath system to house and deliver the biostimulator 100 into the target anatomy. The sheath system can include an inner sheath 206 located within an outer sheath 210. The outer sheath 210 may be slidable over the inner sheath 206. As shown, when the outer sheath 210 is advanced over the inner sheath 206 to an undeployed state, distal ends of the sheaths may be coincident. A shaft system and biostimulator 100 of the biostimulator system 200 can be located within the sheaths. Accordingly, the sheath system can protect the shaft system and biostimulator 100 during delivery into the target anatomy.

Referring to FIG. 4, a side view of a biostimulator system in a deployed state is shown in accordance with an embodiment. The outer sheath 210 can be retracted over the inner sheath 206 to transition the biostimulator system 200 into a deployed state. In a deployed state, a distal portion of the inner sheath 206 can be exposed distal to the outer sheath 210. More particularly, the outer sheath 210 may be advanced over the inner sheath 206 to cover a window 208 of the inner sheath 206 in the undeployed state (see FIG. 3). In the deployed state (continuing in FIG. 4), however, the outer sheath 210 is retracted to expose the window 208. As described below, the window 208 can be an opening in a lateral surface of the inner sheath 206 through which the biostimulator 100 may be deployed.

In an embodiment, the biostimulator transport system 202 includes an input shaft 403 and an output shaft 404. The inner sheath 206 can surround the input shaft 403 and the output shaft 404 to provide a housing for the shafts, as well as to contain a joint connecting the shafts. For example, the joint can include a meshing gear joint, including an input gear 406 and an output gear 408 in a gear train. The shafts may connect at the meshing gear joint. More particularly, the input shaft 403 can extend distally to the joint, e.g., to the input gear 406, and the output shaft 404 can extend proximally from the joint, e.g., from the output gear 408. The input gear 406 and the output gear 408 may be meshed. More particularly, the output gear 408 can be rotationally coupled to the input gear 406 such that rotation of the input shaft 403 drives rotation of the output shaft 404.

The output shaft 404 can extend proximally from the output gear 408 to a biostimulator coupling 410. As described below, the biostimulator coupling 410 can connect the output shaft 404 to the biostimulator 100. For example, the biostimulator 100 may be loaded onto an end of the output shaft 404 at the biostimulator coupling 410, and the biostimulator coupling 410 may rotationally fix the output shaft 404 to the biostimulator 100. Accordingly, rotation of the input shaft 403 can drive rotation of the output shaft 404, which may in turn rotate the biostimulator coupling 410 and the biostimulator 100.

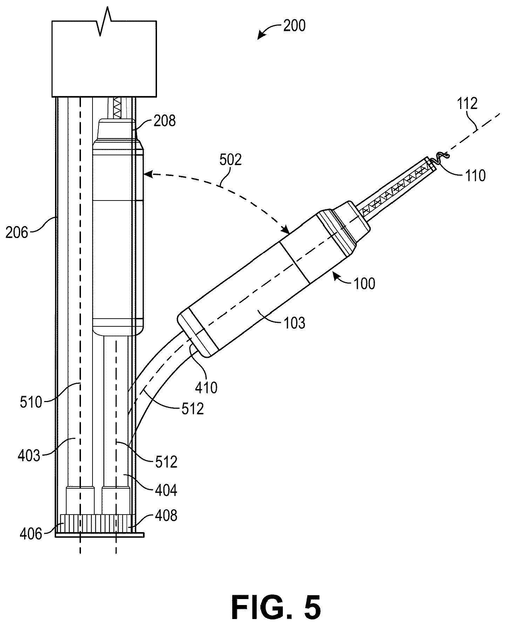

Referring to FIG. 5, a side view of a biostimulator deployed through a window of a biostimulator transport system is shown in accordance with an embodiment. The window 208 of the inner sheath 206 can be located lateral to the biostimulator 100 and the biostimulator coupling 410 on which the biostimulator 100 is mounted. The window 208 can be a cutaway portion of the inner sheath 206. More particularly, the window 208 may be a length of sheath wall that is removed from a location surrounding the biostimulator 100 and the biostimulator coupling 410. The window 208 may have a length and a width that is longer and/or wider than an overall length and width of the biostimulator 100. Accordingly, the window 208 provides a slot through which the biostimulator 100 may pass.

The body 103 of the biostimulator 100 can extend proximally along the longitudinal axis 112 from the biostimulator coupling 410 to the helical fixation element 110. The body 103 and the helical fixation element 110 can be directed or pointed in a proximal, retrograde direction relative to the distal, antegrade direction of the sheaths.

When the biostimulator system 200 is transitioned into the deployed state, the biostimulator 100 can exit the window 208. More particularly, the longitudinal axis 112 can open to an angle 502 from the angular position of the longitudinal axis 112 when the biostimulator system 200 is in the undeployed state. The helical fixation element 110 may therefore be directed proximally (in a retrograde direction) from and at an angle 502 to the distal end of the input shaft 403.

The longitudinal axis 112 of the biostimulator 100 may also be compared and contrasted with axes of the shaft system. More particularly, the input shaft 403 can have an input axis 510 extending longitudinally along the inner sheath 206 through the input gear 406. Similarly, the output shaft 404 may have an output axis 512. The output axis 512 may extend longitudinally along the input sheath and through the output gear 408 when the biostimulator system 200 is in the undeployed state. More particularly, the input axis 510 and the output axis 512 may be parallel to the longitudinal axis 112 of the biostimulator 100 when the biostimulator system 200 is in the undeployed state. Furthermore, the output axis 512 may be coaxially aligned with the longitudinal axis 112 of the biostimulator 100 in the undeployed state. By contrast, when the biostimulator system 200 transitions into the deployed state, the longitudinal axis 112 may extend oblique to the input axis 510. As described below, the portion of the output axis 512 associated with the biostimulator 100 can tilt relative to the input axis 510 and the portion of the output axis 512 associated with the output gear 408. The tilting can occur through bending of the output shaft 404 and/or relative angular movement of the output shaft 404 relative to the input shaft 403. More particularly, the output axis 512 at a location of the biostimulator coupling 410 can extend longitudinally parallel relative to the longitudinal axis 112 of the biostimulator 100. Both the portion of the output axis 512 associated with the biostimulator 100 and the longitudinal axis 112 of the biostimulator 100 may be oblique to the input axis 510, e.g., at the angle 502 to the input axis 510. The gears of the biostimulator system 200 may or may not change relative angular positions, as described below.

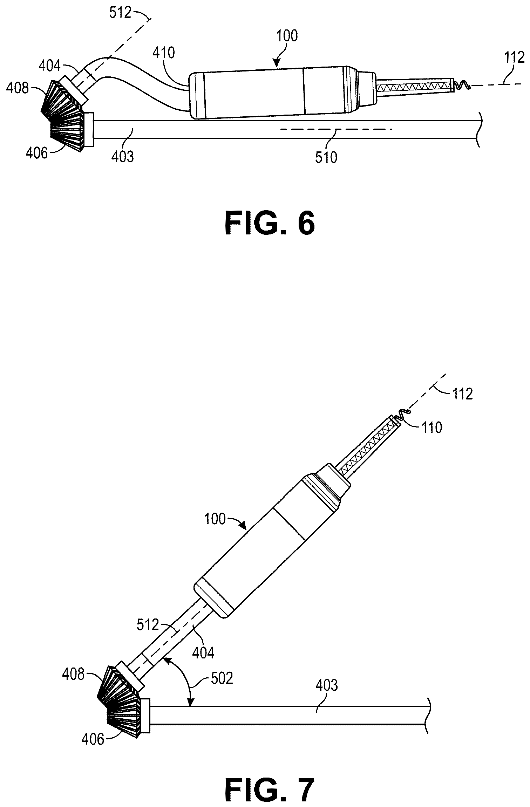

Referring to FIG. 6, a side view of a biostimulator transport system having fixed angle gears in an undeployed state is shown in accordance with an embodiment. The input gear 406 may be meshed to the output gear 408 in the undeployed state. The gear drive may be referred to as a V-drive because the input shaft 403 and the output shaft 404, in a region near the meshed gears, may be parallel to each other and form a V shape having an apex at the joint. In an embodiment, the meshing gear joint includes a fixed angle drive. For example, the input gear 406 and the output gear 408 may include bevel gears such that, when the gears are meshed, the input axis 510 of the input shaft 403 is oblique to the portion of the output axis 512 associated with the output shaft 404. The bevel gears may be conical gears.

Notably, in the undeployed state, the output shaft 404 may flex so that a portion of the output shaft 404 may be positioned parallel to the input shaft 403 of a location near the biostimulator coupling 410. Biostimulator 100 may therefore be held in position against the input shaft 403, when the biostimulator 100 is contained within the inner sheath 206. The sheaths are not shown in FIG. 6, however, it will be appreciated that the outer sheath 210 holds the biostimulator 100 within the inner sheath 206 through the window 208.

Referring to FIG. 7, a side view of a biostimulator transport system having fixed angle gears in a deployed state is shown in accordance with an embodiment. The input gear 406 may be meshed to the output gear 408 in the deployed state. The bevel gears can aid in achieving a desired angle 502 between the shafts. More particularly, when the biostimulator system 200 is transitioned into the deployed state, the output shaft 404 can extend along the portion of the output axis 512 associated with the biostimulator 100 in a direction parallel to the longitudinal axis 112 of the biostimulator 100. For example, when the outer sheath 210 is retracted to uncover the window 208, the biostimulator 100 can be exposed through the window 208 and can be deployed through the window 208. The output shaft 404 can then extend in the retrograde direction, and the retrograde direction can be oblique to the antegrade direction of the input shaft 403.

The output shaft 404 of the fixed angle system may be both flexible and torque transmissive. For example, the output shaft 404 may be formed from a metal (e.g., a shape memory metal) multi-filar coil or braid. The output shaft 404 can therefore be bent into the shape shown in FIG. 6 and recover to the shape shown in FIG. 7. Rotation of the input shaft 403 can transmit torque through the meshing gear joint of the bevel gears. The torque can then be transmitted through the output shaft 404 to the biostimulator coupling 410 to cause rotation of the biostimulator 100 and to screw the helical fixation element 110 into the target tissue 106.

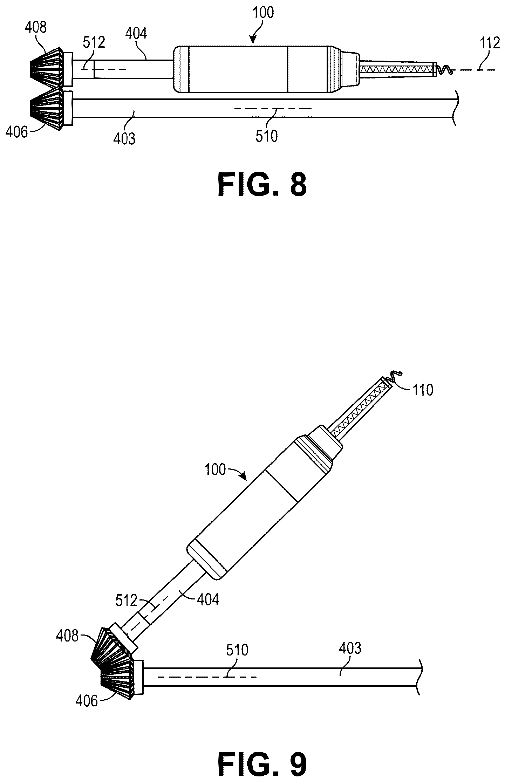

Referring to FIG. 8, a side view of a biostimulator transport system having adjustable angle gears in an undeployed state is shown in accordance with an embodiment. The V-drive may be implemented as an adjustable angle drive. Similar to the embodiment illustrated in FIGS. 6-7, the adjustable angle drive can include bevel gears. The gears of the adjustable angle drive may be movable relative to each other. For example, the output gear 408 may be able to tilt relative to the input gear 406. In the undeployed state, the input gear 406 may not be fully meshed to the output gear 408. For example, the bevel gears may not be meshed along a taper length of the gears. Axes of the gear, which can be coaxial with the input axis 510 and the output axis 512, may be parallel with each other. More particularly, the input shaft 403 can be parallel to the output shaft 404 in the undeployed state. Furthermore, the output shaft 404 can be parallel to the longitudinal axis 112 of the biostimulator 100 in the undeployed state.

Referring to FIG. 9, a side view of a biostimulator transport system having adjustable angle gears in a deployed state is shown in accordance with an embodiment. When the biostimulator 100 deploys through the window 208 to transition the biostimulator system 200 from the undeployed state to the deployed state, the output gear 408 can tilt relative to the input gear 406. The teeth of the gears may tilt into position to mesh with each other. More particularly, the output gear 408 may be meshed to the input gear 406 in the deployed state. Accordingly, in the deployed state, unlike the undeployed state, the input shaft 403 can transmit torque through the engaged gears to the output shaft 404. The torque may therefore be transferred to the biostimulator 100 to screw the helical fixation element 110 into the target tissue 106.

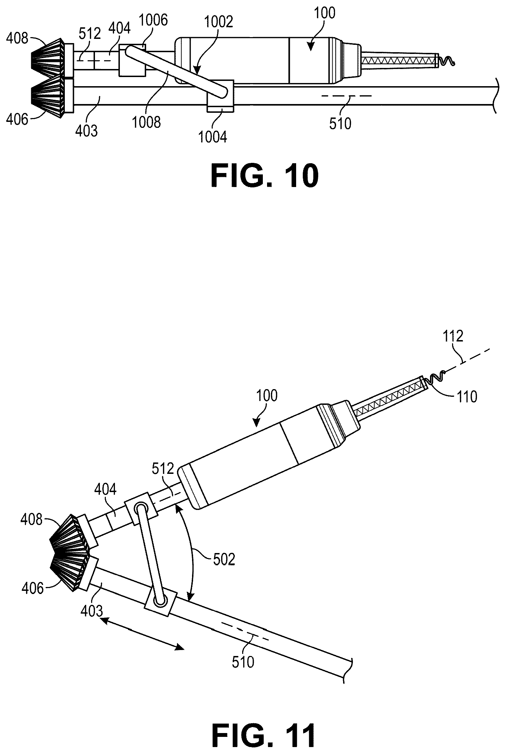

Referring to FIG. 10, a side view of a biostimulator transport system having a shaft actuator in an undeployed state is shown in accordance with an embodiment. The gears of the adjustable angle drive may be tilted into place (to mesh in the deployed state) by shaft actuator 1002. The shaft actuator 1002 can be a deployment mechanism having several components interconnecting the input shaft 403 to the output shaft 404. For example, the shaft actuator 1002 may be coupled to the input shaft 403 at an input collar 1004. Similarly, the shaft actuator 1002 may be coupled to the output shaft 404 at an output collar or hinge 1006. More particularly, the output feature 1006 can be a prismatic joint or a fixed revolute joint in various embodiments. The shaft actuator 1002 can include a collar link 1008 interconnecting the input collar 1004 to the output collar 1006. The collar link 1008 can transmit force from the input collar 1004 to the output collar 1006. Accordingly, pushing or pulling the input collar 1004 along the input shaft 403 can cause movement of the output collar 1006, e.g., sliding or rotation. In the case of a collar, the output collar 1006 can move distally or proximally along the output shaft 404. In the case of a hinge, the output hinge 1006 can rotate relative to the output shaft 404.

Referring to FIG. 11, a side view of a biostimulator transport system having a shaft actuator in a deployed state is shown in accordance with an embodiment. Movement of the shaft actuator 1002 can change the angle 502 between the input shaft 403 and the output shaft 404. As described above, the angle can be an oblique angle between the shaft axes such that the biostimulator 100 is directed outward through the window 208. More particularly, when the shaft actuator 1002 linkage is actuated, the longitudinal axis 112 can tilt outward to direct the helical fixation element 110 toward the target tissue 106. Accordingly, the system can be deployed to tilt the bevel gears into a meshing relationship with each other, and to allow torque to transfer through the shafts to the helical fixation element 110.

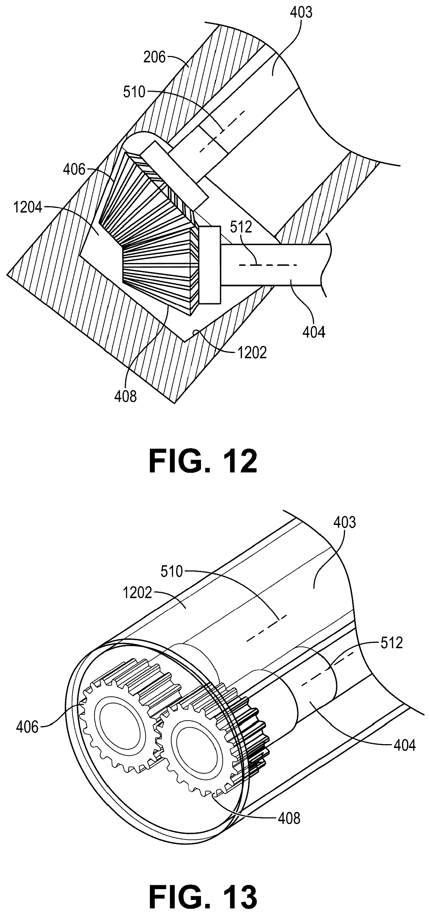

Referring to FIG. 12, a cross-sectional view of gears contained in a boot of a biostimulator transport system is shown in accordance with an embodiment. In each of the fixed angle and adjustable angle embodiments described herein, the input gear 406 and the output gear 408 may mesh within the inner sheath 206. More particularly, the gears can mesh within a boot 1202, which may be a portion of the inner sheath 206. The boot 1202 can have a cavity 1204 containing the input gear 406 and the output gear 408. More particularly, the boot 1202 can define a space of the cavity 1204, which the input gear 406 and the output care can be in a fixed or adjustable relationship within. Accordingly, the gears and the boot 1202 can provide a gearbox having the input shaft 403 to deliver torque into the meshed gears within the boot 1202 and the output shaft 404 can receive and transmit torque out of the meshed gears.

When the input gear 406 is in a fixed relationship with the output gear 408 in the cavity 1204 of the boot 1202, the V-drive system can be a fixed angle system. For the fixed angle system, the boot 1202 may hold the gears together tightly. More particularly, the cavity 1204 may be a space that closely conforms to an outer profile of the meshed gears. The gears can therefore remain in a fixed angle within the boot 1202, and torque may be transmitted through the gear train first along the input shaft 403 in the antegrade direction and second along the output shaft 404 in the retrograde direction. The gears 406, 408 and the boot 1202 form a gearbox.

When the input gear 406 is in an adjustable relationship with the output gear 408 in the cavity 1204 of the boot 1202, the V-drive system can be an adjustable angle system. For the adjustable angle system, the boot 1202 can include a cavity 1204 that is loosely fit to the meshed gears. More particularly, the cavity 1204 can include space for the gears to tilt relative to each other, as described above with respect to FIGS. 8-11. Accordingly, the boot 1202 can hold the gears in the unmeshed, undeployed state, and the gears may tilt into the meshed, deployed state to allow torque to be transmitted through the gearbox via biostimulator coupling 410.

Referring to FIG. 13, a perspective view of meshed spur gears of a biostimulator transport system is shown in accordance with an embodiment. A boot 1202 portion of the inner sheath 206 can contain an alternative embodiment of the fixed angle drive. In an embodiment, the input gear 406 and the output gear 408 are spur gears. The spur gears allow for a 180-degree drive version of the V-drive system, which can transfer torque across a sharp angle. The gears are spur gears such that, when the gears are meshed, the input axis 510 of the input shaft 403 is parallel to the output axis 512 of the output shaft 404. More particularly, the input shaft 403 and the output shaft 404 can remain parallel to each other such that torque can be transmitted down the input shaft 403 in the antegrade direction, and torque can be transmitted up the output shaft 404 in the retrograde direction, 180-degrees to the antegrade direction. Accordingly, when the input torque shaft is rotated at the handle 204, the torque can travel through both gears and turn biostimulator 100 to drive the helical fixation element 110 into the target tissue 106. The output shaft 404 may be flexible to allow the biostimulator 100 to curve outward toward the target tissue 106, as shown in FIG. 5.

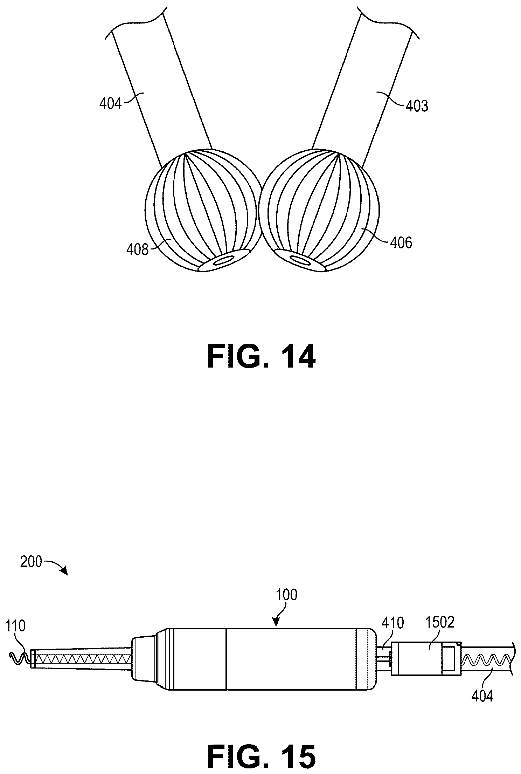

Referring to FIG. 14, a perspective view of meshed spherical gears of a biostimulator transport system is shown in accordance with an embodiment. An alternative embodiment of the angle gear drive can include spherical gears. More particularly, the input gear 406 and the output gear 408 may be spherical gears. Spherical gears can be meshed when the input axis 510 of the input shaft 403 is parallel to the output axis 512 of the output shaft 404. More particularly, unlike the bevel gear embodiment described above, the spherical gears may be meshed in the undeployed state. In the deployed state, when the input axis 510 is oblique to the output axis 512, the spherical gears may also mesh. Accordingly, the spherical gears allow for torque transmission to occur at any angle, e.g., both in the undeployed state and the deployed state.

The biostimulator 100 may detach from the biostimulator transport system 202. For example, an entirety of the output shaft 404 and/or the output gear 408 may disconnect from the biostimulator transport system 202 to allow the biostimulator 100 to be deployed at the target anatomy. Alternatively, only the biostimulator 100, or the biostimulator 100 and the output shaft 404 may disconnect to detach from the biostimulator transport system 202. For example, the biostimulator coupling 410 may include a hex drive that fits into a hex socket of the biostimulator 100. A tether system may be employed to hold the biostimulator 100 against the biostimulator coupling 410. The tether system may release the biostimulator 100, however, to allow the biostimulator 100 to slide in the retrograde direction until the hex drive disengages from the hex socket. In such case, the biostimulator 100 may remain in place with a helical fixation element 110 engaged to the LBB, while the biostimulator transport system 202, including the V-drive system, can be removed from the anatomy.

Torque may be delivered to the input shaft 403 manually or automatically. For example, a user may interact with a knob of the handle 204 to input torque to the input shaft 403. Alternatively, the biostimulator transport system 202 may include a motor to input torque to the V-drive system.

Referring to FIG. 15, a side view of a micromotor of a biostimulator transport system driving a biostimulator is shown in accordance with an embodiment. The biostimulator system 200 can include a micromotor 1502 to drive rotation of the biostimulator 100. In an embodiment, the micromotor 1502 connects directly to the biostimulator 100. More particularly, an output shaft of the micromotor 1502 can connect to a proximal portion of the biostimulator 100. When connected directly to the biostimulator 100, the micromotor 1502 may be mounted on the output shaft 404 of the V-drive system. The joint between the micromotor 1502 and the biostimulator 100 may therefore provide the biostimulator coupling 410. The micromotor 1502 can be driven via an electrical input from the handle 204 to cause the output shaft 404 to rotate the biostimulator 100 and drive the helical fixation element 110 into the target tissue 106.

Optionally, the micromotor 1502 may be indirectly connected to the biostimulator 100. For example, a transmission (not shown) may interconnect the micromotor 1502 to the biostimulator 100. The transmission can receive torque from the output shaft of the micromotor 1502 and deliver torque to the biostimulator 100. By way of example, the transmission can include a gear train for torque reduction to allow a micromotor 1502 having low torque output to drive rotation of the helical fixation element 110 into the target tissue 106. Speed, direction, and torque of the micromotor 1502 motor may also be controlled by power supplied by the handle 204, e.g., via a battery and control system.

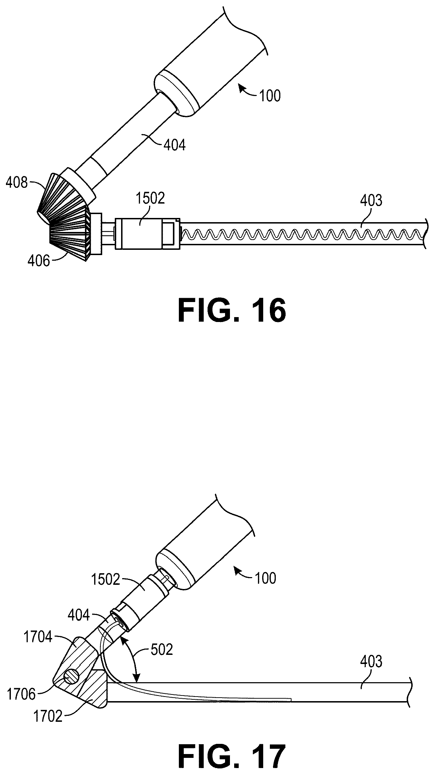

Referring to FIG. 16, a side view of a micromotor driving meshed gears of a biostimulator transport system is shown in accordance with an embodiment. The micromotor 1502 may be mounted on the input shaft 403. The micromotor 1502 can receive electrical input from the handle 204 to generate and transmit torque to the input gear 406. The input gear 406 may mesh with the output gear 408 to transmit the torque to the output shaft 404 and the biostimulator 100.

Referring to FIG. 17, a side view of a micromotor of a biostimulator transport system driving a biostimulator is shown in accordance with an embodiment. Similar to the embodiment shown in FIG. 15, the micromotor 1502 can deliver torque directly to the biostimulator 100. The micromotor 1502 may be mounted on the output shaft 404. In an embodiment, the output shaft 404 is connected to the input shaft 403 via the joint. The joint, however, may not include a gear. More particularly, the joint can include a pivot joint having an input link 1702 connected to the input shaft 403 and an output link 1704 connected to the output shaft 404. The links may rotate relative to each other at a pin 1706. The links can move about the pin 1706 to change the angle 502 between the input shaft 403 and the output shaft 404. Electrical input can be delivered to the micromotor 1502 through a cable to generate the torque that the micromotor 1502 transmits to the biostimulator 100.

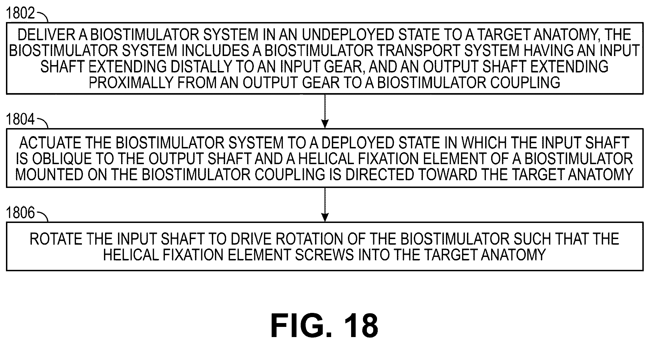

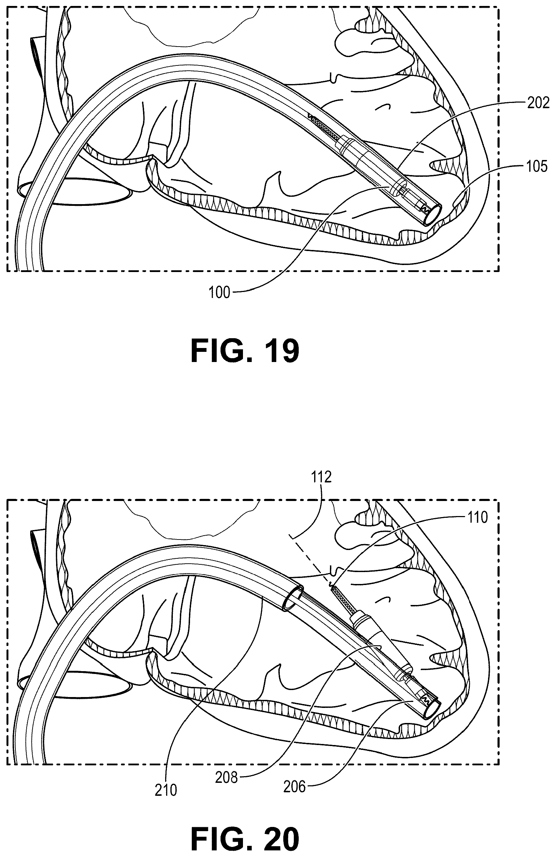

Referring to FIG. 18, a flowchart of an exemplary, non-claimed method of delivering a biostimulator using a biostimulator transport system having rotationally coupled shafts is shown in accordance with an embodiment. FIGS. 19-20 illustrate pictorial views of operations of the method and, thus, FIGS. 18-20 are alternately described below.

Referring to FIG. 19, at operation 1802, the biostimulator system 200 is delivered in the undeployed state to the target anatomy. More particularly, a distal portion of the biostimulator transport system 202, and the biostimulator 100 contained within the system, can be placed in or near the apical space of a heart chamber such as the right ventricle.

Referring to FIG. 20, at operation 1804, the biostimulator system 200 is actuated to the deployed state. The outer sheath 210 can be retracted to expose the window 208 of the inner sheath 206. More particularly, the biostimulator 100 may be exposed through the window 208. The biostimulator 100 can be deployed through the window 208 to the deployed state. In the deployed state, the output shaft 404, which connects to the biostimulator 100, can be oblique to the input shaft 403. The longitudinal axis 112 of the biostimulator 100 is angled outward relative to the input shaft 403. Accordingly, the helical fixation element 110 can be directed to the target anatomy, such as the septal wall 104. The antegrade direction of the longitudinal axis 112 may be opposite to the antegrade direction along which the biostimulator transport system 202 was delivered into the heart chamber.

At operation 1806, the input shaft 403 is rotated to drive rotation of the biostimulator 100. Torque can be applied to the biostimulator 100 via the V-drive system. Torque input to the input shaft 403 may be transmitted through the meshing gear joint. The torque is thereby delivered to the output shaft 404 to rotate the biostimulator 100. Forward pressure may be applied to the biostimulator 100 by pulling on the biostimulator transport system 202 to apply force to the biostimulator 100 in the retrograde direction. The helical fixation element 110 screws into the target anatomy to anchor the biostimulator 100 within the target tissue 106. The biostimulator 100 can detach from the biostimulator transport system 202. The biostimulator transport system 202 may be removed from the patient. Therapeutic pacing impulses may be delivered to the target tissue 106, e.g., the LBB, via an electrode of the biostimulator 100.

The biostimulator transport system 202 having the V-drive mechanism can provide several benefits. A manner in which the delivery system is used when the biostimulator 100 is deployed may mitigate navigational challenges that can be encountered with an antegrade approach. In the antegrade approach, the biostimulator transport system 202 must negotiate an acute bend immediately after crossing the tricuspid valve to place the distal end of the biostimulator 100 at the target location. The acute angle, in addition to the limited space available to maneuver within the right ventricle and variability of cardiac anatomy, can make delivery to the optimal septal wall location difficult. Retrograde delivery, on the other hand, alleviates the navigational challenge. The distal end of the biostimulator system 200 can be easily delivered into the apical space when the biostimulator 100 is deployed outward such that the distal end of the biostimulator 100 is directed toward the septal wall 104, the biostimulator transport system 202 can be easily retracted to pull the helical fixation element 110 against septal wall 104. The helical fixation element 110 can be screwed into the septal wall 104 in the retrograde direction high on the septal wall 104 for optimal capture of a bundle branch. The ease of delivery to the optimal pacing location can make the biostimulator system 200 more accurate and more controllable. Optimal pacing may be more repeatably obtained, and a risk of cardiac perforation may be reduced. Furthermore, as described above, true LBBAP pacing at the bundle branch, rather than at the fascicles or fibers, lower pacing thresholds and extend battery life of the biostimulator 100. The proximal end of the biostimulator 100 may also reside in the apical space post-implant, reducing a risk of interfering with sensitive anatomical structures.

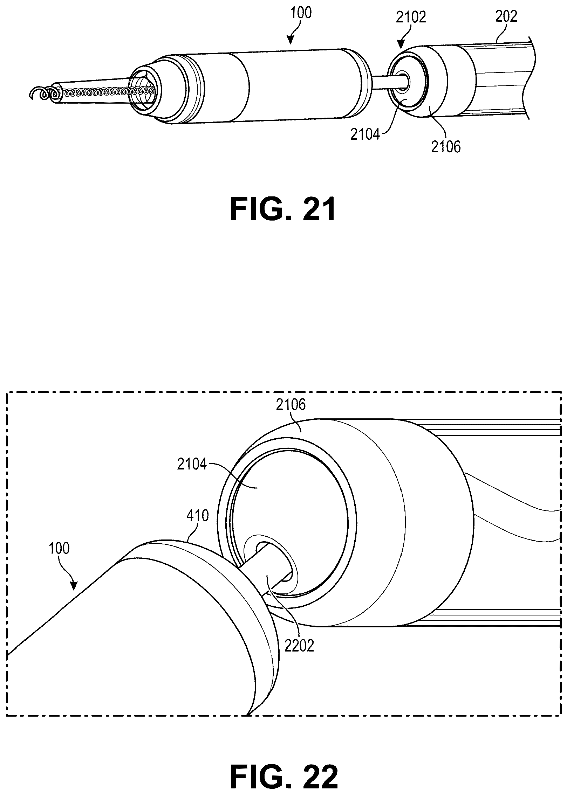

Referring to FIG. 21, a perspective view of a biostimulator transport system including a biostimulator coupling having a universal joint is shown in accordance with an embodiment. The biostimulator system 200 can include a universal joint 2102 having a ball and socket type mechanism 2302 to allow the biostimulator 100 to deflect distally on catheter. The ball and socket type mechanism 2302 can allow freedom of movement of the biostimulator 100 in all directions. Such universal movement may be contrasted with the limited degree of freedom of movement of a pin joint. The universal joint 2102 can include a ball 2104 mounted within a socket 2106. More particularly, a distal tip of the biostimulator system 200 can define the socket 2106 within which the ball 2104 moves.

Referring to FIG. 22, a perspective view of a biostimulator coupling having a universal joint is shown in accordance with an embodiment. The ball 2104 in the socket 2106 is shown in a rotated position relative to FIG. 21. In the rotated position, a central shaft 2202 extends through the ball 2104 at an angle. More particularly, the ball 2104 can include a central channel through which the central shaft 2202 extends. The central shaft 2202 can connect to the biostimulator 100, e.g., at the biostimulator coupling 410. Accordingly, torque delivered through the central shaft 2202 can be transmitted to the biostimulator 100 to screw the helical fixation element 110 into the target tissue 106. It will be appreciated that the ball 2104 can spin and rotate within the socket 2106 to direct the central shaft 2202 in a variety of angles relative to a shaft axis.

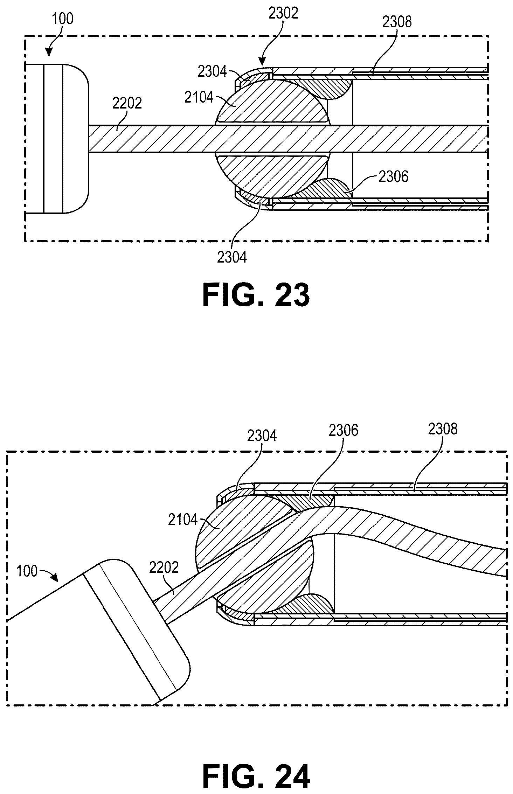

Referring to FIG. 23, a cross-sectional view of a biostimulator coupling having a universal joint in a first state is shown in accordance with an embodiment. The ball and socket mechanism can include a brake, which may be activated to maintain a deflection angle of the central shaft 2202. The brake may include several components that cooperate to squeeze the ball 2104 to restrict movement of the ball 2104. In an embodiment, the brake includes a distal brake sleeve 2304 and a proximal brake sleeve 2306. The brake sleeves 2304, 2306 may be movable relative to each other. For example, one or more pull wires 2308 may be attached to the distal brake sleeve 2304. Retraction of the pull wires 2308, e.g., by a pulling force applied via the handle 204, can move the distal brake sleeve 2304 toward the proximal brake sleeve 2306. A spherical space can be defined between the sleeves, and retraction of the distal brake sleeve 2304 can reduce a size of the space.

The ball 2104 may be metallic or polymeric. For example, the ball 2104 may be formed from stainless steel. Alternatively, the ball 2104 may be formed from a compressible polymer. In either case, the brake can squeeze and bite into the ball surface to restrict movement of the ball 2104 within the socket 2106.

Referring to FIG. 24, a cross-sectional view of a biostimulator coupling having a universal joint in a second state is shown in accordance with an embodiment. The biostimulator 100 may be connected to the biostimulator transport system 202 via a free-rotating ball and socket joint. When the distal brake sleeve 2304 is retracted, surfaces of the brake sleeves 2304, 2306 can apply friction to an outer surface of the ball 2104, locking the ball 2104 in place. A direction of the central shaft 2202, relative to the catheter, can therefore be locked in place. Once the desired angle is achieved and maintained by compressing the ball 2104 between the brake sleeves 2304, 2306, the central shaft 2202 can be rotated to transmit torque to the biostimulator 100. The biostimulator 100 can be pressed against the target tissue 106, and rotation of the biostimulator 100 can drive the helical fixation element 110 into the target tissue 106 to provide therapeutic stimulation and/or pacing.

In the foregoing specification, the invention has been described with reference to specific exemplary embodiments thereof. It will be evident that various modifications may be made thereto without departing from the scope of the invention as set forth in the following claims. The specification and drawings are, accordingly, to be regarded in an illustrative sense rather than a restrictive sense.

## Claims

1. A biostimulator transport system (202), comprising:
an input shaft (403) extending distally to an input gear (406); and
an output shaft (404) extending proximally from an output gear (408) to a biostimulator coupling (410), wherein the output gear (408) is rotationally coupled to the input gear (406) such that rotation of the input shaft (403) drives rotation of the biostimulator coupling (410).

2. The biostimulator transport system of claim 1, further comprising an inner sheath (206) surrounding the input shaft (403) and the output shaft (404), wherein the inner sheath (206) includes a window (208) located lateral to the biostimulator coupling (410).

3. The biostimulator transport system of claim 2, further comprising an outer sheath (210) slidable over the inner sheath (206) such that the outer sheath (210) is advanced to cover the window (208) in an undeployed state and the outer sheath (210) is retracted to expose the window (208) in a deployed state.

4. The biostimulator transport system of claim 2 or 3, wherein the inner sheath (206) includes a boot (1202) having a cavity (1204) containing the input gear (406) and the output gear (408).

5. The biostimulator transport system of claim 4, wherein the input gear (406) is meshed to the output gear (408) in an undeployed state and a deployed state.

6. The biostimulator transport system of claim 4, wherein the output gear (408) tilts relative to the input gear (406) within the cavity (1204) such that the input gear (406) is not meshed to the output gear (408) in an undeployed state and the input gear (406) is meshed to the output gear (408) in a deployed state.

7. The biostimulator transport system of claim 6, further comprising a shaft actuator (1002) coupled to the output shaft (404).

8. The biostimulator transport system of claim 7, wherein movement of the shaft actuator (1002) changes an angle between the input shaft (403) and the output shaft (404).

9. The biostimulator transport system of any one of claims 1 to 8, wherein the input gear (406) and the output gear (408) are spur gears.

10. The biostimulator transport system of claim 9, wherein, when the spur gears are meshed, an input axis (510) of the input shaft (403) is parallel to an output axis (512) of the output shaft (404).

11. The biostimulator transport system of any one of claims 1 to 8, wherein the input gear (406) and the output gear (408) are bevel gears.

12. The biostimulator transport system of claim 11, wherein, when the bevel gears are meshed, an input axis (510) of the input shaft (403) is oblique to an output axis (512) of the output shaft (404).

13. The biostimulator transport system of any one of claims 1 to 8, wherein the input gear (406) and the output gear (408) are spherical gears, wherein the spherical gears are meshed when an input axis (510) of the input shaft (403) is parallel to an output axis (512) of the output shaft (404) and when the input axis (510) is oblique to the output axis (512).

14. A biostimulator system (200), comprising:
the biostimulator transport system (202) of any one of claims 1-13; and
a biostimulator (100) mounted on the biostimulator coupling (410), wherein the biostimulator (100) includes a body (103) having an electronics compartment (120) containing pacing circuitry.

15. The biostimulator system of claim 14, wherein the body (103) extends proximally along a longitudinal axis (112) from the biostimulator coupling (100) to a helical fixation element (110).

## Patentansprüche

1. Biostimulatortransportsystem (202), umfassend:
eine Eingabewelle (403), die sich distal zu einem Eingabezahnrad (406) erstreckt; und
eine Ausgabewelle (404), die sich proximal von einem Ausgabezahnrad (408) zu einer Biostimulatorkopplung (410) erstreckt, wobei das Ausgabezahnrad (408) derart mit dem Eingabezahnrad (406) drehend gekoppelt ist, dass eine Drehung der Eingabewelle (403) eine Drehung der Biostimulatorkopplung (410) antreibt.

2. Biostimulatortransportsystem nach Anspruch 1, ferner umfassend einen inneren Mantel (206), der die Eingabewelle (403) und die Ausgabewelle (404) umgibt, wobei der innere Mantel (206) ein Fenster (208) beinhaltet, das sich seitlich zu der Biostimulatorkopplung (410) befindet.

3. Biostimulatortransportsystem nach Anspruch 2, ferner umfassend einen äußeren Mantel (210), der derart über den inneren Mantel (206) gleitbar ist, dass in einem nicht eingesetzten Zustand der äußere Mantel (210) ausgefahren wird, um das Fenster (208) abzudecken, und in einem eingesetzten Zustand der äußere Mantel (210) eingefahren wird, um das Fenster (208) freizulegen.

4. Biostimulatortransportsystem nach Anspruch 2 oder 3, wobei der innere Mantel (206) einen Schuh (1202) beinhaltet, der einen Hohlraum (1204) aufweist, der das Eingabezahnrad (406) und das Ausgabezahnrad (408) enthält.

5. Biostimulatortransportsystem nach Anspruch 4, wobei das Eingabezahnrad (406) in einem nicht eingesetzten Zustand und einem eingesetzten Zustand mit dem Ausgabezahnrad (408) vernetzt ist.

6. Biostimulatortransportsystem nach Anspruch 4, wobei sich das Ausgabezahnrad (408) innerhalb des Hohlraums (1204) derart in Bezug auf das Eingabezahnrad (406) neigt, dass in einem nicht eingesetzten Zustand das Eingabezahnrad (406) nicht mit dem Ausgabezahnrad (408) vernetzt ist und in einem eingesetzten Zustand das Eingabezahnrad (406) mit dem Ausgabezahnrad (408) vernetzt ist.

7. Biostimulatortransportsystem nach Anspruch 6, ferner umfassend einen Wellenaktor (1002), der mit der Ausgabewelle (404) gekoppelt ist.

8. Biostimulatortransportsystem nach Anspruch 7, wobei eine Bewegung des Wellenaktors (1002) einen Winkel zwischen der Eingabewelle (403) und der Ausgabewelle (404) ändert.

9. Biostimulatortransportsystem nach einem der Ansprüche 1 bis 8, wobei das Eingabezahnrad (406) und das Ausgabezahnrad (408) Stirnräder sind.

10. Biostimulatortransportsystem nach Anspruch 9, wobei, wenn die Stirnräder vernetzt sind, eine Eingabeachse (510) der Eingabewelle (403) parallel zu einer Ausgabeachse (512) der Ausgabewelle (404) ist.

11. Biostimulatortransportsystem nach einem der Ansprüche 1 bis 8, wobei das Eingabezahnrad (406) und das Ausgabezahnrad (408) Kegelräder sind.

12. Biostimulatortransportsystem nach Anspruch 11, wobei, wenn die Kegelräder vernetzt sind, eine Eingabeachse (510) der Eingabewelle (403) schräg zu einer Ausgabeachse (512) der Ausgabewelle (404) ist.

13. Biostimulatortransportsystem nach einem der Ansprüche 1 bis 8, wobei das Eingabezahnrad (406) und das Ausgabezahnrad (408) kugelförmige Zahnräder sind, wobei die kugelförmigen Zahnräder vernetzt sind, wenn eine Eingabeachse (510) der Eingabewelle (403) parallel zu einer Ausgabeachse (512) der Ausgabewelle (404) ist und wenn die Eingabeachse (510) schräg zu der Ausgabeachse (512) ist.

14. Biostimulatorsystem (200), umfassend:
das Biostimulatortransportsystem (202) nach einem der Ansprüche 1-13; und
einen Biostimulator (100), der auf der Biostimulatorkopplung (410) montiert ist, wobei der Biostimulator (100) einen Körper (103) beinhaltet, der ein Elektronikfach (120) aufweist, das eine Schrittmacherschaltung enthält.

15. Biostimulatorsystem nach Anspruch 14, wobei sich der Körper (103) proximal entlang einer Längsachse (112) von der Biostimulatorkopplung (100) zu einem schraubenförmigen Fixierungselement (110) erstreckt.

## Revendications

1. Système de transport de biostimulateur (202), comprenant :
un arbre d'entrée (403) s'étendant de manière distale vers un engrenage d'entrée (406) ; et
un arbre de sortie (404) s'étendant de manière proximale à partir d'un engrenage de sortie (408) vers un raccord de biostimulateur (410), dans lequel l'engrenage de sortie (408) est couplé en rotation à l'engrenage d'entrée (406) de sorte que la rotation de l'arbre d'entrée (403) entraîne la rotation du raccord de biostimulateur (410).

2. Système de transport de biostimulateur selon la revendication 1, comprenant en outre une gaine interne (206) entourant l'arbre d'entrée (403) et l'arbre de sortie (404), dans lequel la gaine interne (206) inclut une fenêtre (208) située latéralement par rapport au raccord de biostimulateur (410).

3. Système de transport de biostimulateur selon la revendication 2, comprenant en outre une gaine externe (210) pouvant coulisser sur la gaine interne (206) de telle sorte que la gaine externe (210) est avancée pour couvrir la fenêtre (208) dans un état non déployé et la gaine externe (210) est rétractée pour exposer la fenêtre (208) dans un état déployé.

4. Système de transport de biostimulateur selon la revendication 2 ou 3, dans lequel la gaine interne (206) inclut un fourreau (1202) ayant une cavité (1204) contenant l'engrenage d'entrée (406) et l'engrenage de sortie (408).

5. Système de transport de biostimulateur selon la revendication 4, dans lequel l'engrenage d'entrée (406) est en prise avec l'engrenage de sortie (408) dans un état non déployé et un état déployé.

6. Système de transport de biostimulateur selon la revendication 4, dans lequel l'engrenage de sortie (408) s'incline par rapport à l'engrenage d'entrée (406) à l'intérieur de la cavité (1204) de telle sorte que l'engrenage d'entrée (406) n'est pas en prise avec l'engrenage de sortie (408) dans un état non déployé et l'engrenage d'entrée (406) est en prise avec l'engrenage de sortie (408) dans un état déployé.

7. Système de transport de biostimulateur selon la revendication 6, comprenant en outre un actionneur d'arbre (1002) couplé à l'arbre de sortie (404).

8. Système de transport de biostimulateur selon la revendication 7, dans lequel le mouvement de l'actionneur d'arbre (1002) modifie un angle entre l'arbre d'entrée (403) et l'arbre de sortie (404).

9. Système de transport de biostimulateur selon l'une quelconque des revendications 1 à 8, dans lequel l'engrenage d'entrée (406) et l'engrenage de sortie (408) sont des engrenages droits.

10. Système de transport de biostimulateur selon la revendication 9, dans lequel, lorsque les engrenages droits sont en prise, un axe d'entrée (510) de l'arbre d'entrée (403) est parallèle à un axe de sortie (512) de l'arbre de sortie (404).

11. Système de transport de biostimulateur selon l'une quelconque des revendications 1 à 8, dans lequel l'engrenage d'entrée (406) et l'engrenage de sortie (408) sont des engrenages coniques.

12. Système de transport de biostimulateur selon la revendication 11, dans lequel, lorsque les engrenages coniques sont en prise, un axe d'entrée (510) de l'arbre d'entrée (403) est oblique par rapport à un axe de sortie (512) de l'arbre de sortie (404).

13. Système de transport de biostimulateur selon l'une quelconque des revendications 1 à 8, dans lequel l'engrenage d'entrée (406) et l'engrenage de sortie (408) sont des engrenages sphériques, dans lequel les engrenages sphériques sont en prise lorsqu'un axe d'entrée (510) de l'arbre d'entrée (403) est parallèle à un axe de sortie (512) de l'arbre de sortie (404) et lorsque l'axe d'entrée (510) est oblique par rapport à l'axe de sortie (512).

14. Système de biostimulateur (200), comprenant :
le système de transport de biostimulateur (202) selon l'une quelconque des revendications 1 à 13 ; et
un biostimulateur (100) monté sur le raccord de biostimulateur (410), dans lequel le biostimulateur (100) inclut un corps (103) ayant un compartiment électronique (120) contenant une circuiterie de stimulation.

15. Système de biostimulateur selon la revendication 14, dans lequel le corps (103) s'étend de manière proximale le long d'un axe longitudinal (112) à partir du raccord de biostimulateur (100) vers un élément de fixation hélicoïdal (110).
